(19) Europäisches Patentamt

European Patent Office

Office européen des brevets

(11) Publication number: **0 248 740 B1**

(12) **EUROPEAN PATENT SPECIFICATION**

(45) Date of publication of patent specification: **17.03.93**  (51) Int. Cl.⁵: **A61K 33/08**, A61K 47/00, A61K 47/02

(21) Application number: **87401283.4**

(22) Date of filing: **05.06.87**

(54) A composition of aluminium hydroxide gel.

(30) Priority: **06.06.86 JP 130352/86**

(43) Date of publication of application:
**09.12.87 Bulletin 87/50**

(45) Publication of the grant of the patent:
**17.03.93 Bulletin 93/11**

(84) Designated Contracting States:
**BE DE FR GB IT**

(56) References cited:
**US-A- 3 364 111**
**US-A- 3 591 680**
**US-A- 4 369 172**

(73) Proprietor: **TERUMO KABUSHIKI KAISHA**
**No. 44-1, Hatagaya 2-chome, Shibuya-ku**
**Tokyo 151(JP)**

(72) Inventor: **Taguchi, Shigeru**
**17-6, 3-chome Tsurumaki Tama-shi**
**Tokyo 206(JP)**
Inventor: **Watanabe, Eiji**
**11-17, 6-chome Kugahara Ota-ku**
**Tokyo 146(JP)**
Inventor: **Yanagawa, Chuji**
**8-1-202, 2-chome, Minamidai**
**Sagamihara-shi Kanagawa-ken 228(JP)**
Inventor: **Hagiwara, Masaru**
**Century Town5-305 185 Taira Miyamae-ku**
**Kawasaki-shi Kanagawa-ken 213(JP)**

(74) Representative: **Gillard, Marie-Louise et al**
**Cabinet Beau de Loménie 55, Rue**
**d'Amsterdam**
**F-75008 Paris (FR)**

EP 0 248 740 B1

# EP 0 248 740 B1

## Description

The present invention relates to a composition of aluminium hydroxide gel whose medical effect is conventionally widely known and, more particularly, to an aqueous suspension formulation of aluminium hydroxide gel having excellent pharmaceutical characteristics for external application.

An aqueous suspension formulation of aluminium hydroxide gel is a medicine or drug listed in the 8th revised edition of Japanese Pharmacopoeia. Its main pharmacological actions include protection of the mucous membrane in the digestive tract, neutralization of gastric juice acidity, and adsorption. The Japanese Pharmacopoeia describes an application of the aqueous suspension formulation of aluminium hydroxide gel for a variety of diseases such as gastric ulcer, duodenal ulcer, hyperacidity, gastritis, dyspeptic chronic diarrhea, and abnormal fermentation in the intestinal tract. Its astringent action for exodermal erosion and associated applications are also reported.

However, since such an aqueous suspension formulation of aluminium hydroxide gel has poor physical stability, it has the following pharmaceutical problems. More specifically, when the aqueous suspension formulation of aluminium hydroxide gel is left to stand, water separates from the gel and forms a supernatant layer. Therefore, at the time of dosage or application, it must be shaken to be rendered homogeneous. The aluminium hydroxide gel changes its viscosity according to the degree of shaking due to its thixotropy. Furthermore, since the gel has a low viscosity compared to other conventional pharmaceutical formulations for external application, the adherence of the medicine applied on an affected part is poor. The medicine tends to be spilled on clothing or bedspreads, thereby soiling them. In this manner, the conventional aqueous suspension formulation of aluminium hydroxide gel has problems in terms of its administration.

The above problems are not specific to the composition of aluminium hydroxide gel but are general in pharmaceutical formulations of aqueous suspensions. Therefore, various pharmaceutical means are known to eliminate such problems and to obtain a homogeneous suspension containing fine particles. For example, a method has been proposed to add sodium alginate, gum arabic, sodium salt of carboxymethyl cellulose (CMC), methyl cellulose or bentonite, to an aqueous medium as a vehicle ("CHOUZAISISIN CHUKAI; YAKUJINIPPON, Ltd.", 1978).

However, such a conventional method is not valid for an aqueous suspension formulation of aluminium hydroxide gel, and a preparation which remains stable for a long period of time has not yet been known.

The present invention has been made in consideration of the above situation and has as its object to provide a pharmaceutical improvement in an aqueous suspension formulation of aluminium hydroxide gel which has a wide range of medical effects as described above.

More specifically, it is an object of the present invention to provide a composition of aluminium hydroxide gel having a sufficient physical stability so that it does not experience phase separation, even if it is left to stand for a long period of time.

In order to achieve the above object of the present invention, there is provided a composition of aluminium hydroxide gel wherein 0.5 to 8.0% by weight (aluminium oxide content) of aluminium hydroxide gel and 0.3 to 8.0% by weight of a cellulose derivative having a hydroxy-alkoxy group are homogeneously dispersed and suspended in water.

According to the present invention, the cellulose derivative having a hydroxy-alkoxy group is at least one member selected from hydroxypropyl-methyl-cellulose, hydroxypropyl-cellulose, and hydroxyethyl-cellulose.

The composition of aluminium hydroxide gel according to the present invention preferably has a viscosity of 100 m Pa.s (cps) or more when measured by a B-type viscometer with rotor No. 1 at 30 rpm and 25°C.

As is apparent from the above description, the important point of the present invention is that the above-mentioned cellulose derivative having a hydroxy-alkoxy group as a stabilizer is added.

The present invention will now be described in detail.

The aluminium hydroxide gel used in the present invention, may be of a medical grade manufactured by a known method. For example, an appropriate amount of an aluminium salt, such as aluminium sulfate, is dissolved in purified water, and an excess amount of a carbonate salt solution, such as a sodium carbonate solution, is dropped into the aluminum salt solution to cause precipitation. The entire reaction system is vigorously mixed by shaking to provide a mixture. The mixture is dialyzed, and the obtained aqueous suspension or its dried powder can be used as the aluminium hydroxide gel.

The stabilizer used in the present invention, i.e., a cellulose derivative having a hydroxy-alkoxy group is semisynthetic cellulose obtained by chemically modifying a cellulose material, such as wood pulp and linter pulp, by a known method. Above all, the following cellulose derivatives are particularly suitable:

(1) hydroxypropyl-methyl-cellulose (to be referred to as HPMC hereinafter)
(2) hydroxypropyl-cellulose (to be referred to as HPC hereinafter)
(3) hydroxyethyl-cellulose (to be referred to as HEC hereinafter)

All of these derivatives are dissolved or swelled in water at room temperature (1 to 30°C). Each derivative can be independently used, or a combination of two or more of these derivatives can be used. The range of substitution or degree of substitution, and the viscosity of these derivatives can vary in relatively wide ranges and are not particularly specified. Generally, the following derivatives are used.

HPMC

* range of substitution:
  methoxy group; 19.0 to 29.0%
  hydroxypropoxy group; 4.0 to 12.0%
* viscosity of 2% aqueous solution: 50 to 30,000 m Pa.s (cps) (20°C)

HPC

* range of substitution:
  hydroxypropoxy group; 53.4 to 77.5%
* viscosity of 2% aqueous solution: 3.0 to 4,000 m Pa.s (cps) (20°C)

HEC

* mean degree of substitution: 1.0 to 1.3
* mean mole of substitution: 1.8 to 2.5
* viscosity of 2% aqueous solution: 20 to 100,000 m Pa.s (cps) (20°C)

The composition of aluminium hydroxide gel of the present invention can be obtained by homogeneously dispersing and suspending in water a predetermined amount of the above-mentioned aluminium hydroxide gel and a predetermined amount of the above-mentioned cellulose derivative having a hydroxy-alkoxy group. The practical method for dispersion and suspension is not particularly specified. For example, suspensions of aluminium hydroxide gel and cellulose derivative can be separately prepared and be mixed by agitation. The cellulose derivative and a predetermined amount of water can be added in the suspension of aluminium hydroxide gel and be mixed by agitation.

The mixing ratio of the aluminium hydroxide gel of the present invention is 0.5 to 8.0% by weight in terms of aluminium oxide content. This range is defined in order to obtain a more sufficient medical effect than required for pharmaceutical stability. In contrast to this, the mixing ratio of the cellulose derivative having a hydroxy-alkoxy group is defined by the requirement for pharmaceutical stability and is 0.3 to 8.0% by weight. When the mixing ratio of the cellulose derivative is less than 0.3% by weight, a sufficient stability cannot be obtained. However, when the mixing ratio of the cellulose derivative is more than 8.0% by weight, the resultant aluminium hydroxide gel can easily become solidified during preservation, rendering dosing or application difficult. In terms of ease in administration or application, it is preferable that, in the range of the composition described above, the viscosity measured by the B-type viscometer with rotor No. 1 at 30 rpm and 25°C is 100 m Pa.s (cps) or more.

Since the aluminium gel composition of the present invention is used as a medicine for external application. Its components must have low toxicity. This will be described.

Aluminium hydroxide gel has been used as an antacid and a covering material for gastric mucosa and thus has low toxicity. Regarding its toxicity, only a minimum toxic dose of 122 g/kg in 4 days in the case of oral administration to a child is known. Regarding the toxicities of HPMC, HEC, and HPC, the following values have been reported:

$LD_{50}$ of HPMC:

* intraperitoneal dosing to a rat; 5,200 mg/kg
* intraperitoneal dosing to a mouse; 5,000 mg/kg MLD (Minimal Lethal Doses) of HEC:
* intraperitoneal dosing to a pregnant rabbit; 500 mg/kg (3 to 7 days)

3

LD$_{50}$ of HPC:

* oral administration to a rat; 10,200 mg/kg

The composition of aluminium hydroxide gel according to the present invention can contain various active drug ingredients in accordance with its application. For example, a composition used for treatment of burned skin effectively contains antibiotics since burned skin can be easily infected with bacterias. The active drug ingredients added for such a purpose are: antibiotics such as oxytetracycline, chlortetracycline, neomycin, gentamycin, and polymixin-B; antihistaminic agents such as diphenhydramine; anesthetics such as benzocaine and lidocaine; antibacterial agents such as povidone iodine, sulfadiazine-Ag, benzalkonium chloride, tolnaftate, and clotrimazole; corticosteroids such as hydroxycortisone, betamethasone valerate, and clobetasol propionate; anti-inflammatory . agents of non-steroids such as indomethacin, flurbiprofen, bufexamac, and bendazac; enzyme preparations such as bromelain, trypsin, and fibrinolysin; anti-malignant tumor agents such as fluorouracil and bleomycin;

The present invention will now be described by way of its examples.

Examples 1 - 3

About 8.5% (A$\ell_2$O$_3$ content) of an aluminium hydroxide gel suspension was prepared, and separately, solutions of HPMC (available from SHIN-ETU CHEMICAL CO., LTD.; trade name "65SH-4000"), HPC (available from NIPPON SODA CO., LTD.; trade name "HPC-M"), and HEC (available from DAICEL CHEMICAL INDUSTRIES, LTD.; trade name "SE850") of predetermined concentrations were prepared.

Subsequently, in accordance with the prescriptions shown in Table 1, the aluminium hydroxide gel suspension was added to each of the solutions of the respective cellulose derivatives. The resultant solutions were mixed by agitation to provide the objective compositions. Note that the concentrations of the respective cellulose derivative solutions were adjusted in accordance with the prescriptions in Table 1.

The compositions obtained in this manner were poured into glass bottles and left in an atmosphere at room temperature (1 to 30°C) for 30 days, and their physical and pharmaceutical stabilities were then tested. The results are also shown in Table 1.

Examples 4 - 6

About 8.3% (A$\ell_2$O$_3$ content) of an aluminium hydroxide gel suspension was prepared. Subsequently, in accordance with the prescriptions shown in Table 1, HPMC (available from SHIN-ETU CHEMICAL CO., LTD.; trade name "90SH-30000") or HEC (available from DAICEL CHEMICAL INDUSTRIES, LTD.; trade name "SE200"), and purified water were added to the suspension so that the total weight was 100 g. The mixture was agitated while being heated to dissolve the added cellulose derivative and cooled, thereby obtaining the objective compositions.

The compositions obtained in this manner were poured into glass bottles and left in an atmosphere at room temperature (1 to 30°C) for 30 days, and their physical and pharmaceutical stabilities were then tested. The results are shown in Table 1.

Comparative Examples 1 - 3

Following the same procedures as in Examples 1 to 3, but using sodium salt of CMC (available from DAICEL CHEMICAL INDUSTRIES, LTD.; trade name "1150"), sodium alginate (available from KAMOGAWA CHEMICAL INDUSTRY, CO., LTD.; trade name "NSPH"), and a carboxyvinyl polymer (available from WAKO PURE CHEMICAL INDUSTRIES, LTD.; trade name "105") in place of the cellulose derivative used in the above examples, compositions of aluminium hydroxide gel were prepared. In Comparative Example 3, a solution of the carboxyvinyl polymer was neutralized by NaOH and then the aluminium hydroxide gel suspension was added.

The above compositions obtained in this manner were poured into glass bottles and left in an atmosphere at room temperature (1 to 30°C) for 30 days, and their physical and pharmaceutical stabilities were then tested. The results are shown in Table 1.

Example 7

8 g of dried aluminium hydroxide gel (4.4 g in A$\ell_2$O$_3$ content) were mixed with a 2% aqueous solution of HEC (available from DAICEL CHEMICAL INDUSTRIES, LTD.; trade name "SP400") so that the total

4

weight was 100g, thereby obtaining an objective composition.

Table 1

| Component (g) | | Example 1 | Example 2 | Example 3 | Example 4 | Example 5 |
|---|---|---|---|---|---|---|
| | Aqueous suspension of aluminium hydroxide gel | 47 | 47 | 47 | 90 | 24.1 |
| | HPMC | 2.0 | | | 0.5 | |
| | HPC | | 2.0 | | | |
| | HEC | | | 1.0 | | 8.0 |
| | CMC–Na | | | | | |
| | Sodium alginate | | | | | |
| | Carboxyvinyl polymer | | | | | |
| | Purified water | 51 | 51 | 52 | 9.5 | 67.9 |
| Remarks | State after left at room temperature for 30 days | Entirely homogeneous semi-solid | Entirely homogeneous semi-solid | Entirely homogeneous semi-solid | Entirely homogeneous semi-solid | Entirely homogeneous viscous liquid |

| Component (g) | Example 6 | Comparative Example 1 | Comparative Example 2 | Comparative Example 3 |
|---|---|---|---|---|
| Aqueous suspension of aluminium hydroxide gel | 48 | 47 | 47 | 47 |
| HPMC | 1.0 | | | 0.5 |
| HPC | | | | |
| HEC | | | | |
| CMC-Na | | 1.0 | | |
| Sodium alginate | | | 1.0 | |
| Carboxyvinyl polymer | | | | 1.0 |
| Purified water | 51.0 | 52 | 52 | 52 |
| Remarks — State after left at room temperature for 30 days | Entirely homogeneous semi-solid | Water separated in upper layer | Water separated in upper layer | Water separated in upper layer |

Clinical applications of the aluminium hydroxide gel according to the present invention will be described.

Clinical Application 1

The composition of aluminium hydroxide gel prepared in accordance with the prescription for Example 1 was externally applied to a decubitus ulcer (1.2 cm × 0.8 cm) at a sacral site of an 80 year-old female

6

patient. The composition could be easily applied without spilling. The affected part was dried as a result of this application, and was cured 7 weeks later.

As is apparent from the results shown in Table 1, the composition of aluminium hydroxide gel according to the present invention has a considerably better physical stability than a conventional one. Water does not separate in the composition even if the composition is preserved for a long period of time. This is attributable to the effective function of the cellulose derivative, added as the stabilizer, as a specially protective colloid, although the exact reason is not clear.

As described above, since the composition of aluminium hydroxide gel of the present invention is homogeneous, physically stable, and water does not separate, its dosage can be accurately determined when it is used for external application. This is a great advantage as a pharmaceutical preparation.

Since the composition of aluminium hydroxide gel of the present invention has an appropriate viscosity and thus good adherence upon application to the skin, it does not flow when it is applied to an affected part. As a result, the composition may not soil the patient's clothing or bedspread, thereby preventing the patient from feeling discomfort. This greatly contributes to an improvement in therapy.

**Claims**

1. A pharmaceutical composition for external application, wherein 0.5 to 8.0% by weight (aluminium oxide content) of aluminium hydroxide gel is homogeneously dispersed and suspended in water, characterized in that 0.3 to 8.0% by weight of at least one cellulose derivative selected from the group consisting of hydroxypropyl-methyl cellulose, hydroxypropyl cellulose and hydroxyethyl cellulose is homogenously dispersed and suspended water, said composition having a viscosity of not less than 100 cps (0.1 Pa.s) when measured by a B-type viscometer with rotor No. 1 at 30 rpm and 25°C."

2. A composition according to claim 1 characterized in that said composition contains an active drug ingredient other than the aluminium hydroxide gel, said active drug ingredient being selected from antibiotics, antihistaminic agents, anesthetics, antibacterial agents, antiinflammatory agents, enzyme preparations and anti-malignant tumor agents.

**Patentansprüche**

1. Arzneimittelzubereitung zum externen Gebrauch, bei welchem 0,5 - 8,0 Gew% (Aluminiumoxidgehalt) Aluminiumhydroxidgel in Wasser homogen dispergiert und suspendiert ist (sind), dadurch gekennzeichnet, daß 0,3 - 8,0 Gew% mindestens eines Cellulosederivats aus der Gruppe Hydroxypropyl-methylcellulose, Hydroxypropyl-Cellulose und Hydroxyethyl-Cellulose in Wasser homogen dispergiert und suspendiert ist (sind), wobei die Zubereitung eine Viskosität, gemessen mit einem Viskosimeter vom Typ B mit einem Rotor Nr. 1 bei 30 Upm und 25°C, von nicht weniger als 100 cps (0,1 Pa•s) aufweist.

2. Zubereitung nach Anspruch 1, dadurch gekennzeichnet, daß sie einen von dem Aluminiumhydroxidgel verschiedenen aktiven Arzneimittelbestandteil, ausgewählt aus Antibiotika, Antihistaminika, Anästhetika, antibakteriellen Mitteln, entzündungswidrigen oder -hemmenden Mitteln, Enzymzubereitungen und Mittel gegen maligne Tumore, enthält.

**Revendications**

1. Composition pharmaceutique à usage externe, dans laquelle 0,5 à 8,0% en poids (teneur en oxyde d'aluminium) d'un gel d'hydroxyde d'aluminium sont dispersés et mis en suspension de façon homogène dans de l'eau, caractérisée en ce que 0,3 à 8,0% en poids d'au moins un dérivé cellulosique sélectionné dans le groupe constitué par l'hydroxypropylméthylcellulose, l'hydroxypropyl-cellulose et l'hydroxyéthylcellulose sont dispersés et mis en suspension de façon homogène dans de l'eau, ladite composition ayant une viscosité supérieure ou égale à 100 cP (0,1 Pa.s) telle que mesurée par un viscosimètre de type B à rotor n° 1 à 30 tr/min et 25°C.

2. Composition selon la revendication 1, caractérisée en ce que ladite composition contient un principe actif médicamenteux autre que le gel d'hydroxyde d'aluminium, ledit principe actif médicamenteux étant sélectionné parmi des antibiotiques, des antihistaminiques, des anesthésiques, des agents antibactériens, des anti-inflammatoires, des préparations enzymatiques et des agents anti-tumeurs malignes.